# FASCICULE DE BREVET EUROPEEN

(11) **EP 2 480 893 B1**
(45) Date de publication et mention de la délivrance du brevet: **27.08.2014**
(21) Numéro de dépôt: 10771161.6
(22) Date de dépôt: 21.09.2010
(51) Int. Cl.: G01N 33/68

(54) **UTILISATION D'ACTIVATEURS DE CANAUX POTASSIQUES K2P EN TANT QU'ANTALGIQUES**
VERWENDUNG VON K2P-KALIUMKANALAKTIVATOREN ALS ANTALGIKA
USE OF K2P POTASSIUM CHANNEL ACTIVATORS AS ANTALGICS

(30) Priorité: 21.09.2009 FR 0956477
(43) Date de publication de la demande: 01.08.2012
(73) Titulaire: Universite d'Auvergne Clermont I, 63000 Clermont Ferrand (FR)
(72) Inventeur: ESCHALIER, Alain, F-63400 Chamalieres (FR); BUSSEROLLES, Jérôme, F-03800 Saulzet (FR); ALLOUI, Abdelkrim, F-63000 Clermont Ferrand (FR); LAZDUNSKI, Michel, F-06000 Nice (FR)
(74) Mandataire: Colombet, Alain André
(86) Numéro de dépôt international: PCT/FR2010/051968
(87) Numéro de publication internationale: WO 2011/033241

(56) Documents cités:
- US-B1- 7 112 403
- ALLOUI ABDELKRIM ET AL: "TREK-1, a K+ channel involved in polymodal pain perception", EMBO JOURNAL, OXFORD UNIVERSITY PRESS, SURREY, GB LNKD- DOI:10.1038/SJ.EMBOJ.7601116, vol. 25, no. 11, 1 juin 2006 (2006-06-01), pages 2368-2376, XP002580938, ISSN: 0261-4189
- ALLOUI A ET AL: "The TREK-1 channel: An attractive target for the development of new analgesics?", DOULEUR ET ANALGESIE 200811 FR LNKD- DOI:10.1007/S11724-008-0108-1, vol. 21, no. 4, novembre 2008 (2008-11), pages 215-220, XP009133127, ISSN: 1011-288X
- DUPRAT F ET AL: "The neuroprotective agent riluzole activates the two P domain K+ channels TREK-1 and TRAAK", MOLECULAR PHARMACOLOGY, AMERICAN SOCIETY FOR PHARMACOLOGY AND EXPERIMENTAL THERAPEUTICS, US, vol. 57, no. 5, 1 mai 2000 (2000-05-01), pages 906-912, XP002256646, ISSN: 0026-895X
- NOËL JACQUES ET AL: "The mechano-activated K+ channels TRAAK and TREK-1 control both warm and cold perception.", THE EMBO JOURNAL 6 MAY 2009 LNKD- PUBMED:19279663, vol. 28, no. 9, 6 mai 2009 (2009-05-06), pages 1308-1318, XP002580940, ISSN: 1460-2075

## Description

La présente invention concerne le traitement et la prévention de la douleur. Plus particulièrement, la présente invention démontre l'implication des canaux potassiques K2P dans l'effet antalgique de la morphine. La présente invention fournit donc une méthode de criblage pour l'identification d'antalgiques.

La douleur peut se définir comme une expérience sensorielle et émotionnelle désagréable liée à une lésion tissulaire existante ou potentielle ou décrite en termes d'une telle lésion. En pratique, la prise en charge de la douleur perçue par le patient résulte de l'interaction entre le phénomène qui génère la douleur, les capacités de l'individu à l'intégrer, et la réponse des professionnels en situation de la reconnaître et de la traiter.

Au moins 50 % des patients adultes hospitalisés souffrent de la douleur (Durieux et al., 2001 Presse Med. 30:572-6). L'intensité de la douleur post-opératoire peut être un facteur de risque de la persistance d'une douleur au-delà de cette période (Basbaum et al., 1999 Curr Biol. 9:R429-31) et son contrôle efficace est associé à une réduction des complications post-opératoires. Les douleurs chroniques rebelles sont source d'altérations majeures de la qualité de vie, d'incapacité et de handicaps. Elles induisent une consommation importante de soins ainsi que de nombreux arrêts de travail. Dans une grande enquête européenne, un cinquième des sujets interrogés par téléphone ont déclaré souffrir de douleurs chroniques, pour lesquelles 85% d'entre eux précisent avoir consulté au cours des six derniers mois, au moins une fois un médecin. Ainsi, dans sa pratique quotidienne, tout médecin se voit confronté à de tels patients et ce nombre va augmenter vu le vieillissement de la population. La douleur est également le symptôme le plus fréquent lié au cancer. Elle concerne de 30 à 50% des patients cancéreux tous stades confondus, et de 65 à 90 % des patients à un stade avancé. La très grande majorité des patients interviewés souffrant de douleurs chroniques indiquent recevoir un traitement antalgique médicamenteux. Les médicaments sur prescription les plus souvent cités sont les anti-inflammatoires et les opioïdes faibles, alors que les opioïdes forts le sont avec une fréquence de l'ordre de 5%. Concernant les médicaments en vente libre, les médicaments mentionnés sont les anti-inflammatoires et le paracétamol, et dans une moindre mesure les opioïdes faibles dans les pays où ils peuvent être obtenus sans prescription (Breivik et al., 2006 Eur J Pain. 10:287-333).

La prise en charge médicamenteuse des douleurs reste problématique avec des médicaments dont les ratios bénéfice/risque, en fonction des patients (susceptibilité individuelle aux traitements, sujets âgés, enfants, *etc*.), ne sont pas toujours en faveur des effets bénéfiques. Les innovations thérapeutiques ne sont pas fréquentes et les produits anciens sont encore utilisés en première intention dans de nombreuses indications.

Les antalgiques opioïdes regroupent la morphine et l'ensemble des dérivés hémi-synthétiques ou synthétiques de cet alcaloïde. La morphine reste le produit de référence dans le traitement des douleurs par excès de nociception intenses, notamment les douleurs post-opératoires et les douleurs cancéreuses. Les opioïdes présentent des effets antalgiques mais aussi psychodysleptiques, dépresseurs respiratoires, émétiques, cardiovasculaires, sur la musculature lisse et sur le système immunitaire. Les effets indésirables liés à l'utilisation des opioïdes se caractérisent par de la constipation presque systématique, des nausées et vomissements chez plus de la moitié des patients, une sédation fréquente, le risque de dépression respiratoire nécessitant une surveillance, et des hallucinations ou confusion chez le sujet âgé qui, bien que rares, nécessitent une réduction des doses. De plus, les effets constipants constituent un handicap majeur en particulier chez la personne âgée et en cancérologie.

La morphine produit ses effets via l'activation de récepteurs opioïdes, dont on connaît trois sous-types (µ, δ et κ). Les effets thérapeutiques (analgésique) et indésirables (par exemple la constipation et la dépression respiratoire) impliquent préférentiellement l'activation du récepteur µ (Roy et al., 1998 Brain Res Mol Brain Res. 56:281-3). La volonté de dissocier ces effets impose donc de travailler en aval de ce récepteur. Bien que le mécanisme d'action exact ne soit pas connu, la liaison de la morphine au récepteur p entraîne l'activation de protéines Gi_{/0}, l'inhibition de l'adénylate cyclase, l'activation de la phospholipase C, et, conséquemment, l'inhibition de canaux calcium voltage dépendants ainsi que l'activation de canaux potassium (Zuo, 2005 Anesth. Analg. 101 :728-34). Parmi les canaux potassium, deux canaux à rectification entrante, les canaux GIRK et les canaux dépendants de l'ATP (K_{ATP}), ont été décrits comme étant impliqués, pour partie, dans l'effet antalgique de la morphine (Ocana et al., 2004 Eur J Pharmacol. 500:203 19). L'état de la technique et notamment le document US 7,112,403 décrit une méthode d'identification de substances ayant des propriétés anesthésiques afin de produire chez un mammifère un état d'inconscience réversible avec une amnésie simultanée et un effet analgésique lors de son inhalation, par l'utilisation des protéines de transport de potassium dans laquelle une activation du transport du potassium est indicative que la substance à identifier a des propriétés anesthésiques. L'état de la technique Alloui et al., 2008 Doul. et Analg. 21:215-220 identifie les canaux potassiques TREK-1 comme cible pour la découverte de nouveaux composés antalgiques. Cependant, l'implication potentielle d'autres canaux potassium, tels que les canaux potassiques K2P, dans l'effet antalgique de la morphine n'a pas été décrite à ce jour.

Afin de pallier les effets indésirables de la morphine, l'industrie pharmaceutique développe actuellement soit des antalgiques opioïdes sans action sur les récepteurs µ, soit des antagonistes périphériques des récepteurs µ qui ne s'opposent cependant qu'aux effets constipants, et pas aux effets dépresseurs respiratoires. Toutefois, les premiers risquent d'avoir une efficacité antalgique moindre que celle des agonistes des récepteurs µ, et les seconds doivent être co-prescrits avec les opioïdes.

Il serait donc souhaitable d'obtenir un antalgique aussi efficace que la morphine, mais dénué d'effets constipant et dépresseur respiratoire, deux effets indésirables majeurs en termes respectivement de fréquence et de gravité.

### DESCRIPTION DE L'INVENTION

Les inventeurs démontrent pour la première fois, l'implication des canaux potassiques K2P, et plus particulièrement de TREK-1 et de TRAAK, dans l'effet antalgique de la morphine. Ainsi, la délétion du gène codant TREK-1 ou TRAAK conduit à la suppression de l'effet antalgique de l'opiacé. Par ailleurs, la déplétion du gène codant TREK-1 ou TRAAK ne réduit pas l'effet constipant et/ou dépresseur respiratoire de la morphine. Les canaux TREK-1 et TRAAK sont donc nécessaires à l'action antalgique mais ne participent pas ces effets indésirables. Les canaux potassiques K2P de la famille TREK/TRAAK peuvent donc être utilisés comme cibles pour cribler de nouveaux antalgiques aussi efficaces que la morphine avec un meilleur ratio bénéfice/risque.

La présente invention fournit donc une méthode de criblage pour isoler de nouveaux antalgiques efficaces et bien tolérés, et permet par conséquent la synthèse, la validation, le développement et la formulation des antalgiques ainsi sélectionnés.

### Utilisation des canaux potassiques K2P en tant que cibles

Les canaux potassiques TREK-1 et TRAAK étant impliqués dans l'effet antalgique de la morphine mais pas dans ses effets indésirables, les canaux potassiques K2P de la famille TREK/TRAAK sont utiles comme cibles lors de criblages pour identifier des composés antalgiques. Les composés antalgiques ainsi identifiés sont caractérisés en ce qu'ils ne présentent pas d'effet constipant et/ou dépresseur respiratoire.

On décrit donc une méthode de criblage pour l'identification d'un composé antalgique comprenant les étapes suivantes :
a) identifier un activateur d'un canal potassique K2P par criblage de composés candidats;
b) mesurer l'effet antalgique dudit activateur; et,
c) mesurer l'effet constipant et/ou dépresseur respiratoire dudit activateur.

La méthode selon l'invention peut comporter l'étape supplémentaire de sélection d'un composé antalgique dénué d'effet constipant et/ou dépresseur respiratoire.

Par « *composé antalgiques* », on entend ici un composé capable d'atténuer ou d'abolir la douleur.

Par « *canal patassique K2P* », on entend ici la famille UniProt n° TC 1.A.1.8 (appelée aussi « two pore domain potassium channel family »). Le canal potassique K2P selon l'invention est choisi parmi les membres de la famille TREK/TRAAK, plus particulièrement parmi TREK-1, TREK-2 et TRAAK.

Par « *canal TREK-1* », on entend ici un canal potassique K2P comprenant au moins une sous-unité dont la séquence est montrée dans l'entrée Swiss-Prot n° 095069 (SEQ ID NO : 1) ou dont la séquence est dérivée de cette séquence. De préférence, le canal TREK-1 est un canal constitué de deux sous-unités de séquences choisies parmi la sequence SEQ ID NO : 1 et ses séquences dérivées. Le canal TREK-1 peut par exemple être un canal homodimérique constitué de deux sous-unités de séquence SEQ ID NO : 1.

Par « *canal TREK-2* », on entend ici un canal potassique K2P comprenant au moins une sous-unité dont la séquence est montrée dans l'entrée Swiss-Prot n° P57789 (SEQ ID NO : 2) ou dont la séquence est dérivée de cette séquence. De préférence, le canal TREK-2 est un canal constitué de deux sous-unités de séquences choisies parmi la séquence SEQ ID NO : 2 et ses séquences dérivées. Le canal TREK-2 peut par exemple être un canal homodimérique constitué de deux sous-unités de séquence SEQ ID NO : 2.

Par « *canal TRAAK* », on entend ici un canal potassique K2P comprenant au moins une sous-unité dont la séquence est montrée dans l'entrée Swiss-Prot n°Q9NYG8 (SEQ ID NO : 3) ou dont la séquence est d érivée de cette séquence. De préférence, le canal TRAAK est un canal constitué de deux sous-unités de séquences choisies parmi la séquence SEQ ID NO : 3 et ses séquences dérivées. Le canal TRAAK peut par exemple être un canal homodimérique constitué de deux sous-unités de séquence SEQ ID NO : 3.

Les « *séquences dérivées* » incluent notamment les variants d'épissage, les variants alléliques, et les séquences homologues dans des espèces mammifères non humaines. Les séquences dérivées incluent notamment des séquences au moins 50, 60, 70, 80, 85, 90, 95 ou 99 % identiques à l'une des séquences SEQ ID NO : 1, SEQ ID NO : 2 ou SEQ ID NO : 3.

Par « *séquence d'acides aminés au moins 95%* (par exemple) *identique à une séquence de référence »,* on entend une séquence identique à la séquence de référence si ce n'est que cette séquence peut comporter jusqu'à cinq mutations (substitutions, délétions et/ou insertions) pour chaque partie de cent acides aminés de la séquence de référence. Ainsi, pour une séquence de référence faisant 100 acides aminés, un fragment de 95 acides aminés et une séquence de 100 acides aminés comportant 5 substitutions par rapport à la séquence de référence sont deux exemples de séquences 95% identiques à la séquence de référence. Le pourcentage d'identité est généralement déterminé en utilisant un logiciel d'analyse de séquences. Les séquences d'acides aminés à comparer sont alignées pour obtenir le maximum de degré d'identité. A cette fin, il peut être nécessaire d'introduire de manière artificielle des espaces (« gaps ») dans la séquence. Une fois l'alignement optimal réalisé, le degré d'identité est établi par enregistrement de toutes les positions pour lesquelles les acides aminés des deux séquences comparées sont identiques, par rapport au nombre total de positions. Le programme « needle », qui fait appel à l'algorithme d'alignement global « Needleman-Wunsch » pour trouver l'alignement optimal (avec gaps) de deux séquences sur la totalité de leur longueur, peut par exemple être utilisé. Ce programme est notamment disponible sur le site ebi:ac.uk.

Les séquences dérivées peuvent différer de la séquence de référence par substitution, délétion et/ou insertion d'un ou de plusieurs acides aminés, et ceci à des positions telles que ces modifications ne portent pas significativement atteinte à l'activité biologique des peptides. Les substitutions peuvent notamment correspondre à des substitutions conservatives.

Dans un mode de réalisation particulier, la séquence des dérivés diffère de la séquence SEQ ID NO: 1,2 où 3 uniquement par la présence de substitutions conservatives. Les substitutions conservatives sont des substitutions d'acides aminés de même classe, telles que des substitutions d'acides aminés aux chaînes latérales non chargées (tels que l'asparagine, la glutamine, la serine, la cystéine, et la tyrosine), d'acides aminés aux chaînes latérales basiques (tels que la lysine, l'arginine, et l'histidine), d'acides aminés aux chaînes latérales acides (tels que l'acide aspartique et l'acide glutamique), d'acides aminés aux chaînes latérales apolaires (tels que l'alanine, la valine, la leucine, l'isoleucine, la proline, la phénylalanine, la méthionine et le tryptophane).

Par *« activateur d'un canal potassique K2P»,* on entend ici un composé capable d'augmenter l'activité biologique d'un canal potassique K2P. Contrairement à la morphine, les activateurs décrits n'ont pas pour effet d'activer les récepteurs opioïdes. De ce fait, ils sont dépourvus d'effet constipant et/ou dépresseur respiratoire. De préférence, les activateurs agissent directement sur le canal potassique K2P, c'est-à-dire qu'ils interagissent directement avec ledit canal. Les activateurs peuvent également activer le canal potassique K2P via une cascade de signalisation. Dans ce second cas, les activateurs agissent nécessairement en aval des récepteurs opioïdes, c'est-à-dire qu'ils modulent l'activité d'un composant de la cascade qui est situé en amont du canal potassique K2P, mais en aval des récepteurs opioïdes. Le fait qu'un activateur du canal potassique K2P agit en aval des récepteurs opioïdes peut aisément être testé en comparant l'activité du canal potassique K2P en présence et en l'absence d'un inhibiteur des récepteurs opioïdes tel que par exemple la naloxone. Le fait que l'activité du canal potassique K2P est la même en présence et en l'absence de l'inhibiteur des récepteurs opioïdes indique que l'activateur agit en aval des récepteurs opioïdes.

Des méthodes pour déterminer si un composé est capable d'augmenter l'activité biologique d'un canal potassique K2P sont bien connues de l'homme du métier.

Il est par exemple possible d'identifier un activateur d'un canal TREK-1 en utilisant la méthode décrite par Alloui et al. (EMBO J. 2006 25:2368-76). Une telle méthode peut par exemple comprendre les étapes suivantes:
- fournir une cellule exprimant un canal TREK-1 (par exemple un neurone DRG) ;
- mesurer le courant en l'absence du composé candidat ; et
- mesurer le courant en présence du composé candidat ;
où une augmentation du courant en présence du composé candidat indique que le composé candidat est un activateur du canal TREK-1. Ledit courant peut par exemple être induit par un composé tel que l'acide arachidonique ou par une acidification intracellulaire. Plus particulièrement, les activateurs du canal TREK-1 sont capables de produire une augmentation de l'intensité du courant de neurones de ganglions rachidiens dorsaux provenant de souris sauvages lorsque l'intensité du courant est évaluée par la technique dite de « patch-clamp en condition cellules entières » dans les conditions décrites dans Alloui et al. (EMBO J. 2006 25:2368-76).

Il est également possible d'identifier un activateur d'un canal TREK-1 en utilisant la méthode décrite par Duprat et al. (Mol. Pharmacol. 2000, 57 :906-912). Dans cette méthode, des systèmes d'expression hétérologues (par exemple des cellules COS) sont transfectés avec le gène codant TREK-1, puis les courants sont mesurés par la technique dite de « patch-clamp en condition cellules entières ».

Les méthodes décrites dans Alloui et al. (EMBO J. 2006 25:2368-76) et dans Duprat et al. (Mol. Pharmacol. 2000, 57 :906-912) sont également applicables à l'étude d'autres canaux potassiques K2P que TREK-1, en remplaçant les animaux invalidés pour TREK-1 par des animaux invalidés pour le canal que l'on souhaite étudier (par exemple TREK-2 ou TRAAK).

Alternativement, la méthode de criblage peut être mise en oeuvre en utilisant une plateforme de criblage à haut-débit adaptée au criblage de modulateurs de canaux potassiques (Falconer et al., 2002 J Biomol Screen. 7:460-5 ; Ford et al., 2002 Prog Drug Res. 58:133-68).

Les composés antalgiques ainsi identifiés sont caractérisés en ce qu'ils ne présentent pas d'effet constipant et/ou dépresseur respiratoire. Par ailleurs, ils peuvent également être dépourvus d'autres effets indésirables.

Par composé présentant un « *effet antalgique* », on entend ici un composé capable d'atténuer ou d'abolir la douleur. L'effet antalgique d'un composé peut être mesuré par n'importe quelle méthode bien connue de l'homme du métier, par exemple par le test de sensibilité mécanique de von Frey ou par le test thermique d'immersion de la queue dans l'eau chaude (voir Exemples 1 et 2).

Par composé présentant un « *effet constipant* », on entend ici un composé capable de diminuer la défécation. L'effet constipant d'un composé peut être mesuré par n'importe quelle méthode bien connue de l'homme du métier, par exemple en utilisant une méthode globale de recueil des fèces (voir Exemples 1 et 4).

Par composé présentant un « *effet dépresseur respiratoire »,* on entend ici un composé capable de diminuer la fréquence respiratoire. L'effet dépresseur respiratoire d'un composé peut être mesuré par n'importe quelle méthode bien connue de l'homme du métier, par exemple en mesurant la fréquence respiratoire grâce à un pléthysmographe barométrique (voir Exemples 1 et 5).

Par composé « *dépourvu d'effet constipant et*/*ou dépresseur respiratoire* », on entend un composé en présence duquel la défécation et/ou la fréquence respiratoire est supérieure ou égale à 70%, 80 %, 90 % ou 95 % de la valeur observée en l'absence dudit composé. De préférence, la défécation et/ou la fréquence respiratoire n'est pas diminuée de manière statistiquement significative en présence dudit composé. Les effets constipant et/ou dépresseur respiratoire étant dose dépendant, l'absence d'effet constipant et/ou dépresseur respiratoire d'un composé antalgique est de préférence évaluée à une dose équianalgésique à celle à laquelle la morphine produit un effet antalgique.

Dans le cadre de ces méthodes de criblage, les composés candidats peuvent par exemple correspondre à des ligands naturels d'un canal potassique K2P, des molécules chimiques, des aptamères, des peptides et des anticorps. Dans un mode de réalisation préféré, les composés candidats sont de petites molécules chimiques (« small molecules »).

Dans un mode de réalisation particulier, on décrit une méthode de criblage pour l'identification d'un composé antalgique comprenant les étapes suivantes :
a) fournir un composé candidat;
b) déterminer si ledit composé candidat active un canal potassique K2P;
où la détermination que ledit composé candidat active ledit canal potassique K2P indique que ledit composé candidat est un composé antalgique

Cette méthode comprend en outre l'étape de mesurer l'effet antalgique dudit composé candidat qui active ledit canal potassique K2P, et/ou l'étape de mesurer l'effet constipant et/ou dépresseur respiratoire dudit composé activant ledit canal potassique K2P.

La méthode selon l'invention peut comporter l'étape supplémentaire de sélection d'un composé antalgique dénué d'effet constipant et/ou dépresseur respiratoire.

L'utilisation d'un canal potassique K2P comme cible pour identifier un composé antalgique lors d'un criblage de composés candidats est également décrite. Plus précisément, une telle utilisation vise à l'identification d'activateurs de canaux potassique K2P, qui possèdent un effet antalgique.

Les méthodes de criblage selon l'invention peuvent être réalisées *in vitro* ou *in vivo.* Lorsque ces méthodes impliquent des expériences sur des animaux modèles tels que des rats ou des souris, ces animaux sont sacrifiés à l'issue de ces méthodes. Ainsi, ces méthodes peuvent comprendre une étape supplémentaire de sacrifice des animaux modèles ayant éventuellement été utilisés.

### Utilisation thérapeutique d'activateurs de canaux potassiques K2P

Etant donné que l'invalidation des canaux TREK-1 et TRAAK conduit à la diminution des effets antalgiques de la morphine sans affecter les effets indésirables tels l'effet constipant et/ou dépresseur respiratoire, les activateurs des canaux TREK-1 et TRAAK sont des antalgiques dépourvus de ces effets indésirables de la morphine. On décrit également un activateur d'un canal potassique K2P de la famille TREK/TRAAK pour une utilisation dans le traitement ou la prévention de la douleur. De tels activateurs sont particulièrement avantageux pour traiter ou prévenir la douleur puisqu'ils sont dépourvus d'effet constipant et/ou dépresseur respiratoire.

La douleur peut être de diverses natures. Elle peut être aiguë ou chronique. Il peut par exemple s'agir d'une douleur post-opératoire, d'une douleur associée à une crise hyperalgique, d'une douleur associée à un cancer, d'une douleur ostéoarticulaire, d'une douleur viscérale ou d'une douleur neuropathique, ladite douleur survenant à toute période de la vie. Dans un mode de réalisation préféré, il s'agit d'une douleur chronique, et plus particulièrement d'un syndrome douloureux chronique tel que la fibromyalgie, de l'intestin irritable ou d'une douleur *sine materia.* Dans un autre mode de réalisation préféré, il s'agit d'une douleur intestinale, stomacale ou pulmonaire, liée ou non à un cancer, telle qu'une douleur associée à un cancer colorectal, à un cancer du colon, à un cancer de l'estomac, à la colite ulcéreuse, à la maladie inflammatoire chronique de l'intestin (désignée par l'abréviation « MICI » ou « IBD »), à l'intestin irritable, à un cancer du poumon ou à l'asthme.

La douleur peut être traitée à tout stade. Par « *fraitement* », on entend traitement à titre curatif (visant à au moins soulager, freiner ou stopper la douleur). Par « *prévention* », on entend traitement à titre prophylactique (visant à réduire le risque d'apparition de la douleur).

Les activateurs peuvent par exemple correspondre à des ligands naturels d'un canal potassique K2P, à des molécules chimiques, à des aptaméres, à des peptides, ou à des anticorps.

L'activateur peut par exemple correspondre à l'un des activateurs de canaux potassiques K2P déjà connus dans l'art. Ainsi, les canaux de la famille TREK/TRAAK sont ouverts de manière réversible par les lysophospholipides qui possèdent de larges têtes polaires (lysophosphatidylcholine, lysophosphatidylinositol) et par des acides gras polyinsaturés (acide linolénique, acide arachidonique) (Fink et al., 1998 EMBO J. 17:3297-308). Des esters de l'acide caféique, notamment le cinnamyl 1-3,4-dihydroxy-α-cyanocinnamate (CDC) et l'acide caféique phenetyl ester (CAPE), augmentent le courant TEK-1 sur cellules bovines adréno-fasciculaires (Danthi et al., 2004 Mol Pharmacol. 65:599-610). Enfin, des fénamates tels que l'acide flufénamique, l'acide niflumique et l'acide méfénamique activent les canaux TREK-1, TREK et TRAAK dans des systèmes d'expression hétérologue (Takahira et al., 2005 Pflugers Arch. 451:474-8). Les composés spécifiquement mentionnés ci-dessus sont des exemples d'activateur.

De ce fait, l'activateur peut être par exemple choisi parmi un lysophospholipide, un acide gras polyinsaturé, un ester de l'acide caféique ou un fénamate.

Alternativement, les activateurs de canaux potassiques K2P peuvent être isolés par les méthodes de criblage décrites ci-dessus dans le paragraphe intitulé «Utilisation des canaux potassiques K2P en tant que cibles».

On décrit également une méthode de manufacture d'un médicament contenant un activateur d'un canal potassique K2P comprenant les étapes; suivantes :
a) produire ledit médicament;
b) mesurer l'effet antalgique dudit médicament; et, optionnellement,
c) mesurer l'effet constipant et/ou dépresseur respiratoire dudit médicament.

Une telle méthode de manufacture, qui permet de tester la qualité de différents lots de production, est utile lors de la production industrielle de médicaments. Plus particulièrement, cette méthode permet de vérifier qu'un lot donné de médicament contenant un activateur d'un canal potassique K2P présente un effet antalgique, et ne présente pas d'effet constipant et/ou dépresseur respiratoire. A l'étape (a), le médicament peut être produit par n'importe quelle méthode connue de l'homme de l'art. La production d'un tel médicament comprend typiquement la synthèse de l'activateur d'un canal potassique K2P, puis sa formulation en un produit pharmaceutique.

Le médicament peut être destiné à être administré par n'importe quelle voie appropriée, par exemple orale, sous-linguale, nasale, buccale, transdermique, intraveineuse, sous-cutanée, intramusculaire et/ou rectale. Dans un médicament, le principe actif (c'est-à-dire l'activateur selon l'invention) est combiné à un véhicule pharmaceutiquement acceptable (c'est-à-dire tout solvant, milieu de dispersion, agent retardant l'absorption, etc., qui ne produit pas de réaction secondaire, par exemple allergique, chez l'humain ou l'animal). Les véhicules pharmaceutiquement acceptables sont bien connus de l'homme de l'art, et incluent ceux décrits dans « Remington's Pharmaceutical Sciences » (Mack Publishing Company, Easton, USA, 1985).

On décrit également une méthode de traitement ou de prévention de la douleur, comprenant l'administration d'une quantité thérapeutiquement efficace d'un activateur d'un canal potassique K2P à un individu en ayant besoin. L'individu est de préférence un mammifère, plus particulièrement un humain. La dose thérapeutique efficace peut facilement être déterminée par l'homme du métier.

On décrit en outre l'utilisation d'un activateur d'un canal potassique K2P pour la préparation d'un médicament pour traiter ou prévenir la douleur.

Les exemples et figures suivants illustrent l'invention sans en limiter la portée.

### DESCRIPTION DES FIGURES

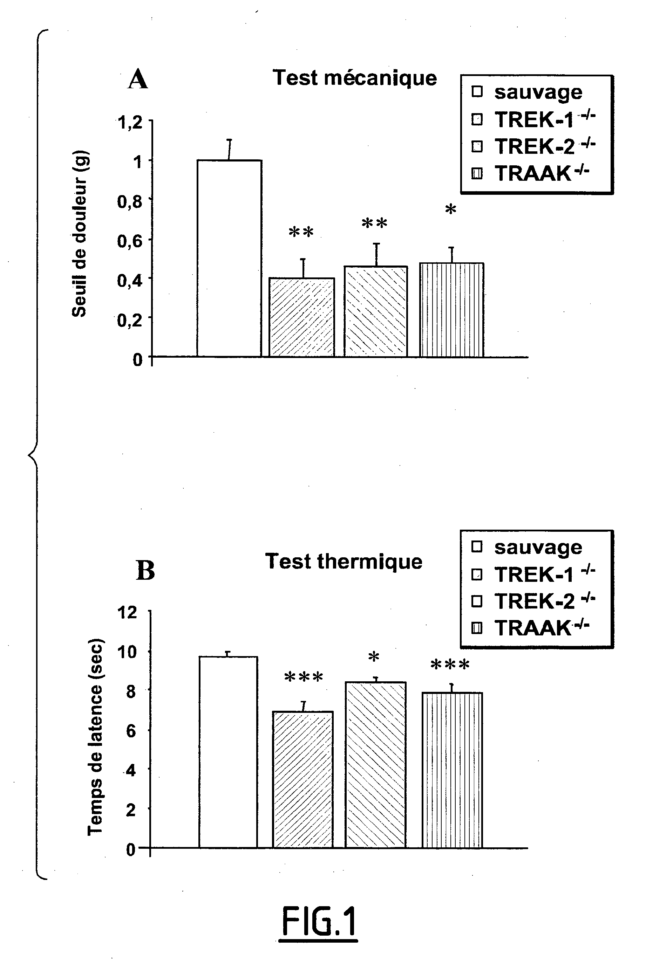

Figure 1 : A l'état basal, les animaux knock-out (KO) montrent une hypersensibilité au test mécanique de von Frey **(A)** ou au test thermique **(B)** par rapport aux animaux sauvages (WT) (WT vs KO : test de Student-t : *, P<0.05, **, P<0.01, ***, P<0.001, n=8/15 par groupe).

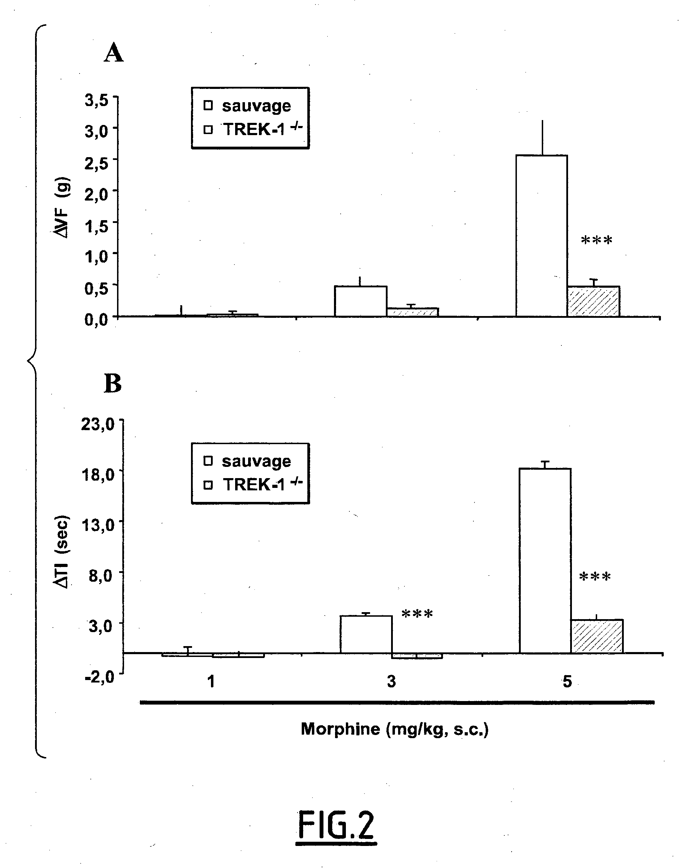

Figure 2 : Effet dose de morphine dans le test mécanique de von Frey **(A)** ou le test thermique d'immersion de la queue dans l'eau à 46°C **(B)** chez les animaux sauvages et TREK-1^{-/-}. Les résultats sont exprimés en variations de seuils nociceptifs par rapport aux valeurs des souris de même génotype traitées par le véhicule. Quel que soit le test utilisé, l'analyse statistique montre globalement un effet-dose de la morphine (F=153.3, P<0.001 ; F=17.5, P<0.001) et un effet génotype (F=143.2, P<0.001 ; F=14.2, P<0.001). L'effet antalgique de la morphine est diminué chez les souris TREK-1^{-/-} par rapport aux animaux sauvages (Anova à deux voies, post hoc Student Newman Keuls ; ***, P<0.001, n=6/9 par dose et par génotype).

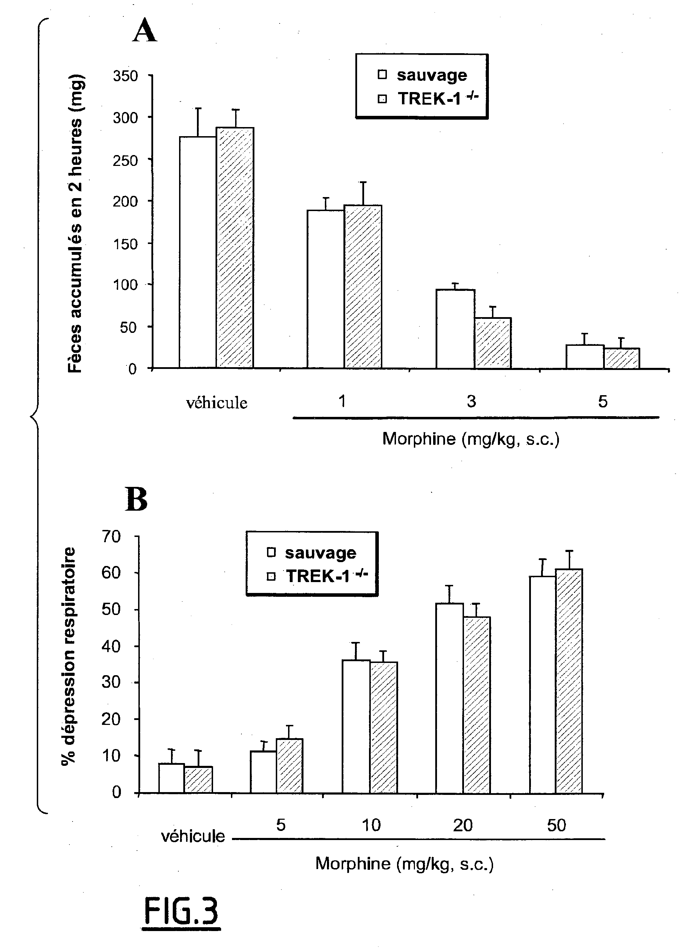

Figure 3 : **(A)** Quantité de fèces accumulés en deux heures suivant l'injection de véhicule ou de morphine (1 ; 3 ; ou 5 mg/kg, s.c.) chez les animaux sauvages et TREK-1^{-/-}. L'analyse statistique montre globalement un effet-dose de la morphine (P<0.001) mais pas d'effet du génotype (n=5/6 par dose et génotype). **(B)** Dépression respiratoire induite par des doses croissantes de morphine (5 ; 10 ; 20 ou 50 mg/kg, s.c.) chez les animaux sauvages et TREK-1^{-/-}. L'analyse statistique montre globalement un effet-dose de la morphine (P<0.001) mais pas d'effet du génotype (n=6/8 par dose et génotype).

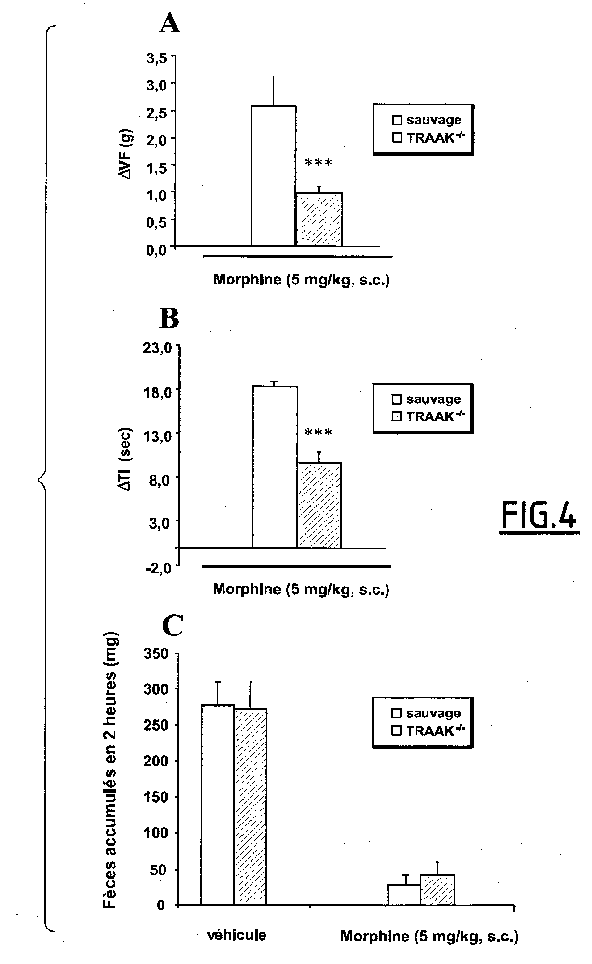

Figure 4 : Effet antinociceptif de la morphine (5mg/kg, s.c.) dans le test mécanique de von Frey **(A)** et le test thermique d'immersion de la queue dans l'eau à 46°C **(B)** chez les animaux sauvages et TRAAK^{-/-}. Les résultats sont exprimés en variations de seuils nociceptifs par rapport aux valeurs des souris de même génotype traitées par le véhicule. Quel que soit le test utilisé, l'analyse statistique montre que l'effet antalgique de la morphine est diminué chez les souris TRAAK^{-/-} par rapport aux animaux sauvages (test de Student-t : ***, P<0.001, n=12/14 par groupe). **(C)** Quantité de fèces accumulés en deux heures suivant l'injection de véhicule ou de morphine (5 mg/kg, s.c.) chez les animaux sauvages et TRAAK^{-/-} (n=6 par dose et génotype).

### DESCRIPTION DES SEQUENCES DU LISTAGE DE SEQUENCES

SEQ ID NO : 1 correspond à la séquence d'une sous-unité d'un canal TREK-1.
SEQ ID NO : 2 correspond à la séquence d'une sous-unité d'un canal TREK-2.
SEQ ID NO : 3 correspond à la séquence d'une sous-unité d'un canal TRAAK.

### EXEMPLES

### Exemple 1 : Protocoles

### Animaux

Des souris mâles C57B1/6J (Charles River Lab, France), TREK-1^{-/-}, TREK-2^{-/-} et TRAAK^{-/-} (IPMC, Nice Sophia Antipolis), pesant de 20 à 30 grammes, ont été placées en cage avec nourriture et eau *ad libitum* en environnement thermo régulé à 22°C avec un cycle jour/nuit de 12h/12h. Les déta ils concernant la génération des animaux KO ont été décrits précédemment (Heurteaux et al., 2004 EMBO J. 23:2684-95; Guyon et al., 2009 J. Neurosc. 29:2528-33). Les expérimentations ont été réalisées en aveugle dans une pièce calme par le même expérimentateur en prenant soin de respecter les exigences réglementaires sur l'expérimentation animale.

### Molécules

Les molécules suivantes ont été utilisées : chlorhydrate de morphine (Cooper, Melun, France), chlorhydrate de naloxone (Sigma Chemical Co., St Louis, MO). Les solutions ont été préparées extemporanément dans du NaCl (0,9%). La naloxone (1 mg/kg, s.c.) a été administrée 15 minutes avant l'injection de morphine ou de véhicule.

### Tests de nociception

Les tests utilisés comprenaient des tests utilisant des stimuli thermiques (test d'immersion de la queue dans l'eau chaude à 46°C) et mécaniques (sensibilité mécanique au test des filaments de von Frey). *La sensibilité thermique* est mesurée par le test d'immersion de la queue dans de l'eau à la température nociceptive de 46°C (Janssen et al., 1963 Arzneimittelforschung. 13:502-7). L'animal est maintenu manuellement et la queue est immergée dans un bain-marie jusqu'au retrait de celle-ci par l'animal ou jusqu'au « cut off », fixé à 30 s. Le seuil basal (pré-traitement) est défini par la moyenne des deux premiers temps de latence ne différant pas de plus d'une seconde. Les animaux ont été habitués à la contention pendant une semaine avant le début de l'expérience. *La sensibilité mécanique* est mesurée par application de filaments de Von Frey (Bioseb) de force croissance (0,01 à 2 g) sous les pattes de l'animal (Tal and Bennett, 1994 Pain. 57:375-82). Les animaux sont placés dans des boîtes (85 x 35 mm, séparation opaque entre les souris, fond grillagé pour accéder à la voûte plantaire) 20 minutes avant le test, pour les habituer. Les filaments sont appuyés perpendiculairement sous la patte arrière droite des souris jusqu'à ce qu'ils se courbent. Cette opération est répétée cinq fois, avant de passer au filament suivant dans le cas où aucune réaction n'a été obtenue. Lorsque la pression exercée correspond au seuil de sensibilité tactile de l'animal, celui-ci a une réaction de retrait de la patte équivalent à un battement réflexe. La valeur de pression du filament est alors retenue en tant que valeur seuil.

### Transit intestinal

L'évaluation du transit intestinal a été réalisée en utilisant une méthode globale de recueil des fèces en cinétique pendant 2 heures suivant l'injection de morphine ou de véhicule (NaCl 0.9%). Les animaux sont placés dans des boîtes (85 x 35 mm, séparation opaque entre les souris, fond grillagé pour le recueil des fèces) 20 minutes avant le test, pour habituation. Les fèces sont recueillis, et pesés immédiatement après recueil, toutes les heures pendant 2 heures suivant l'injection de morphine ou de véhicule.

### Mesure de la fréquence respiratoire

La fréquence respiratoire a été mesurée de manière non invasive (Drorbaugh and Fenn, 1955 Pediatrics. 16:81-7; Matthes et al., 1998 J Neurosci. 18:7285-95) en utilisant un pléthysmographe barométrique (Emka Technologies, VA, USA) comportant 8 chambres permettant la mesure en parallèle sur plusieurs animaux. Le logiciel IOX (Emka Technologies, VA, USA) a été utilisé pour le calcul de la fréquence respiratoire. Chaque dose de morphine ou de véhicule a été administrée, et leurs effets sur la fréquence respiratoire évalués chez quatre souris sauvages et quatre souris TREK-1^{-/-} en parallèle. Les animaux ont été habitués aux chambres pendant 15 min avant l'injection. L'analyse de la fréquence respiratoire pendant cette période de 15 minutes a révélé une fréquence respiratoire stable. La moyenne de la fréquence respiratoire pendant cette période a donc été utilisée pour normaliser l'effet de la morphine ou du véhicule sur la fréquence respiratoire pendant la période du test (90 minutes).

### Analyses statistiques

Les données expérimentales ont été analysées en utilisant le logiciel Sigma STAT, version 3.0 pour Windows (STAT32 Software Inc., San Diego, California).

### Exemple 2 : Rôle des canaux TREK-1, TREK-2 et TRAAK dans la physiologie de la nociception

Quel que soit le test utilisé, test de sensibilité mécanique de von Frey (Fig. 1 A) ou test thermique d'immersion de la queue dans l'eau chaude à 46°C (Fig.1B), il a été montré que les animaux TREK-1^{-/-} ou TRAAK^{-/-} ont des seuils de douleur significativement plus faibles que les animaux sauvages. Il a également été montré que les seuils de douleur des animaux TREK-2^{-/-} sont significativement plus faibles que ceux des animaux sauvages (Fig.1A et 1B).

### Exemple 3 : Rôle des canaux TREK-1 dans l'action antalgique de la morphine

Les animaux TREK-1^{-/-} ayant des seuils de douleur, avant injection de morphine ou de véhicule, significativement plus bas que ceux des animaux sauvages (Fig.1A et 1B), les différences entre les valeurs pré- et post-injection ont été calculées pour chaque animal, puis comparées.

L'analyse statistique des données, utilisant une Anova à deux voies, a fait apparaître des effets principaux pour la morphine (F= 153,3; p < 0,001; *F* = 17,5; p < 0,001) et le génotype (*F* = 143,2; *p* < 0,001; F = 14,2; p < 0,001), respectivement pour les tests thermique et mécanique. De plus, alors que la morphine augmente les temps de latence du retrait de la queue dans l'eau chaude à 46°C et les seuils de douleur mécanique dans les deux génotypes, ces effets antalgiques de la morphine sont très significativement diminués chez les animaux TREK-1^{-/-} par rapport aux animaux sauvages (Fig.2A et 2B).

En conclusion, ceci démontre que les canaux TREK-1 sont impliqués dans l'effet antalgique de la morphine.

### Exemple 4 : Influence de la délétion des canaux TREK-1 sur la constipation induite par la morphine

Les récepteurs opioïdes sont des acteurs clés dans l'inhibition du transit gastro-intestinal induite par la morphine (Reisine and Pasternak, 1996, « The Pharmacological Basis of Therapeutics », Editeurs : Hardman JG, Gilman AG, and Limbird, pages 521-555).

L'impact de la morphine sur le transit d'animaux TREK-1^{-/-} a donc été étudié dans l'objectif d'évaluer l'influence de la délétion sur cet effet indésirable. Les effets de la morphine sur les fonctions gastro-intestinales ont été évalués par la mesure de la production de fèces récoltés toutes les heures pendant 2 heures après injection de morphine ou du véhicule. Pour s'assurer que les deux génotypes ne différent pas dans leur consommation de nourriture et d'eau, cette consommation a été mesurée et normalisée par animal (les cages contenant de 3 à 5 animaux) et les données moyennées pour 3 cages par génotype.

En l'absence de traitement par la morphine, aucune différence n'a été observée (consommation de nourriture : animaux sauvages, 3,17 ± 0,23; TREK-1^{/-}, 3,21 ± 0,14 g/animal/24 h ; consommation d'eau : animaux sauvages, 3,69 ± 0,41; TREK-1^{-/-}, 4,03 ± 0,05 mL/animal/24 h). Le traitement par le véhicule a donné un profil de production fécale identique dans les deux génotypes, suggérant que les deux génotypes ne diffèrent pas dans leur fonction gastro-intestinale en condition normale. La morphine induit une diminution de la défécation comparable dans les deux génotypes lors de la période testée par rapport au traitement avec le véhicule (Fig.3A).

Cette expérience démontre que les canaux TREK-1 ne sont pas impliqués dans l'effet constipant de la morphine.

### Exemple 5 : Influence de la délétion des canaux TREK-1 sur la dépression respiratoire induite par la morphine

Parmi les effets indésirables de la morphine, le plus délétère est certainement la dépression respiratoire, qui peut entraîner la mort dans les cas de surdosage. La dépression respiratoire induite par la morphine se produit également via l'activation des récepteurs opioïdes (Santiago and Edelman, 1985 J Appl Physiol. 59:1675-85; Reisine and Pasternak, 1996, « The Pharmacological Basis of Therapeutics », Editeurs : Hardman JG, Gilman AG, and Limbird, pages 521-555), de plus les souris dont le gène codant le récepteur µ a été invalidé sont protégées vis-à-vis de cet effet indésirable (Matthes et al., 1998 J Neurosci. 18:7285-95; Dahan et al., 2001 Anesthesiology. 94:824-32; Romberg et al., 2003 Br J Anaesth. 91:862-70). Afin de déterminer si la dépression respiratoire induite par la morphine est altérée par la délétion du gène codant les canaux TREK-1, la fréquence respiratoire des animaux sauvages et TREK-1^{-/-} a été mesurée en utilisant un pléthysmographe barométrique après l'injection de morphine ou de véhicule.

La fréquence respiratoire basale n'est pas différente chez les deux génotypes. De plus, quel que soit le génotype, ni le véhicule ni la morphine à la dose de 5 mg/kg n'influencent la fréquence respiratoire. En revanche, quel que soit le génotype, l'administration de morphine aux doses 10, 20 et 50 mg/kg entraîne une diminution de la fréquence respiratoire dose dépendante sans différence entre les génotypes (Fig. 3B).

La délétion du gène qui code pour TREK-1 ne réduit pas l'effet dépresseur respiratoire de la morphine. Le canal TREK-1 n'est donc pas en cause dans cet effet indésirable.

### Exemple 5 : Rôle des canaux TRAAK dans l'action antalgique et l'effet constipant de la morphine

Afin de tester l'hypothèse de l'implication des canaux TRAAK dans l'action antalgique de la morphine, des souris sauvages et knock-out pour les canaux TRAAK (TRAAK^{-/-}) ont été utilisées, et les effets de la morphine à la dose de 5 mg/kg injectée par voie sous- cutanée on été mesurés.

Quel que soit le test utilisé, test de sensibilité mécanique de von Frey ou test thermique d'immersion de la queue dans l'eau chaude à 46°C, il a été trouvé que les animaux TRAAK^{-/-} ont des seuils de douleur, avant injection de morphine ou de véhicule, significativement plus bas que ceux des animaux sauvages (Fig.1A et 1B).

Les valeurs après l'injection de morphine ou de véhicule ont ensuite été mesurées, et les différences entre les valeurs pré- et post-injection ont été calculées pour chaque animal. L'analyse statistique des données, utilisant un test de Student-t a fait apparaître que l'effet antalgique de la morphine est significativement diminué chez les animaux TRAAK^{-/-} par rapport aux animaux sauvages (Fig. 4A et 4B).

Ce résultat montre que les canaux TRAAK sont également impliqués dans l'effet antalgique de la morphine.

La constipation induite par la morphine chez les animaux TRAAK^{-/-} a également été étudiée. Les effets de la morphine sur les fonctions gastro-intestinales ont été évalués par la mesure de la production de fèces récoltés toutes les heures pendant 2 heures après injection de morphine ou de véhicule. Pour s'assurer que les deux génotypes ne diffèrent pas dans leur consommation de nourriture et d'eau, cette consommation a été mesurée et normalisée par animal (les cages contenant de 3 à 5 animaux) et les données moyennées pour 3 cages par génotype.

En l'absence de traitement par la morphine, aucune différence n'a été observée (consommation de nourriture : animaux sauvages, 3,17 ± 0,23; TRAAK^{-/-}, 3,58 ± 0,52 g/animal/24 h ; consommation d'eau : animaux sauvages, 3,69 ± 0,41; TRAAK^{-/-}, 4,25 ± 0,48 mL/animal/24 h). Le traitement par le véhicule a donné un profil de production fécale identique dans les deux génotypes, suggérant que les deux génotypes ne diffèrent pas dans leur fonction gastro-intestinale en condition normale. La morphine induit une diminution de la défécation comparable dans les deux génotypes lors de la période testée par rapport au traitement avec le véhicule (Fig.4C).

Ce résultat démontre que les canaux TRAAK ne sont pas impliqués dans l'effet constipant de la morphine.

## Revendications

1. Méthode de criblage pour l'identification d'un composé antalgique dépourvu d'effet constipant et/ou dépresseur respiratoire comprenant les étapes suivantes :
a) fournir un composé candidat;
b) déterminer si ledit composé candidat active un canal potassique choisi parmi TREK-1, TRAAK et TREK-2;
comprenant en outre l'étape de mesurer l'effet antalgique dudit composé candidat activant ledit canal potassique, in vivo sur des animaux modèles non humains, et l'étape de mesurer l'effet constipant et/ou dépresseur respiratoire dudit composé activant ledit canal potassique, in vivo sur des animaux modèles non humains.

2. Méthode de criblage selon la revendication 1, comprenant en outre la sélection d'un composé antalgique dénué d'effet constipant et/ou dépresseur respiratoire.

3. Méthode de criblage pour l'identification d'un composé antalgique dépourvu d'effet constipant et/ou dépresseur respiratoire comprenant les étapes suivantes :
a) identifier un activateur d'un canal potassique choisi parmi TREK-1, TRAAK et TREK-2 par criblage de composés candidats;
b) mesurer l'effet antalgique dudit activateur; et
c) mesurer l'effet constipant et/ou dépresseur respiratoire dudit activateur.

4. Méthode de criblage selon la revendication 3, comprenant en outre la sélection d'un composé antalgique dénué d'effet constipant et/ou dépresseur respiratoire.

5. Utilisation d'un canal potassique choisi parmi TREK-1, TRAAK et TREK-2 comme cible pour identifier un composé antalgique dépourvu d'effet constipant et/ou dépresseur respiratoire lors d'un criblage de composés candidats.

6. Utilisation selon la revendication 5, où ledit composé antalgique est un activateur dudit canal potassique.

7. Méthode de criblage selon l'une quelconque des revendications 1 à 4, ou utilisation selon la revendication 5 ou 6, où lesdits composés candidats sont choisis parmi des ligands naturels dudit canal potassique, des molécules chimiques, des aptamères, des peptides et des anticorps.

## Patentansprüche

1. Verfahren zum Screenen für die Identifikation einer schmerzlindernden Verbindung, die keine verstopfende und/oder Atem dämpfende Wirkung aufweist, folgende Schritte umfassend:
a) Liefern einer Kandidatenverbindung
b) Bestimmen, ob die Kandidatenverbindung einen Kaliumkanal, ausgewählt aus TREK-1, TRAAK und TREK-2, aktiviert;
außerdem den Schritt des Messens der schmerzlindernden Wirkung der den Kaliumkanal aktivierenden Kandidatenverbindung, in vivo an nicht menschlichen Modelltieren, und den Schnitt des Messens der verstopfenden und/oder Atem dämpfenden Wirkung der den Kaliumkanal aktivierenden Verbindung, in vivo an nichtmenschlichen Modelltieren, umfassend.

2. Verfahren zum Screenen nach Anspruch 1, außerdem die Auswahl einer schmerzlindernden Verbindung, der die verstopfende und/oder Atem dämpfende Wirkung fehlt, umfassend.

3. Verfahren zum Screenen für die Identifikation einer schmerzlindernden Komponente, die keine verstopfende und/oder Atem dämpfende Wirkung aufweist, folgende Schritte umfassend:
a) Identifizieren Aktivators eines Kaliumkanals, ausgewählt aus TREK-1, TRAAK und TREK2 durch Screenen von Kandidatenverbindungen;
b) Messen der schmerzlindernden Wirkung des Aktivators; und
c) Messen der verstopfenden und/oder Atem dämpfenden Wirkung des Aktivators.

4. Verfahren zum Screenen nach Anspruch 3, außerdem die Auswahl einer schmerzlindernden Verbindung, der die verstopfende und/oder Atem dämpfende Wirkung fehlt, umfassend.

5. Verwendung eines Kaliumkanals, ausgewählt aus TREK-1, TRAAK und TREK-2, als Ziel zum Identifizieren einer schmerzlindernden Verbindung, die keine verstopfende und/oder Atem dämpfende Wirkung aufweist, bei einem Screening von Kandidatenverbindungen.

6. Verwendung nach Anspruch 6, bei der die schmerzlindernde Verbindung ein Aktivator des Kaliumkanals ist.

7. Verfahren zum Screenen nach einem beliebigen der Ansprüche 1 bis 4 oder Verwendung nach Anspruch 5 oder 6, bei denen die Kandidatenverbindungen ausgewählt sind aus natürlichen Liganden des Kaliumkanals, chemischen Molekülen, Aptameren, Peptiden und Antikörpern.

## Claims

1. A screening method for identifying an antalgic compound without any constipating and/or respiratory depressive effect, comprising the following steps:
a) providing a candidate compound;
b) determining whether said candidate compound activates a potassium channel selected from TREK-1, TRAAK and TREK-2;
further comprising the step for measuring the antalgic effect of said candidate compound activating said potassium channel, *in vivo* on non-human model animals, the step for measuring the constipating and/or respiratory depressive effect of said compound activating said potassium channel, *in vivo* on non-human model animals.

2. The screening method according to claim 1, further comprising the step of selecting an antalgic compound without any constipating and/or respiratory depressive effect.

3. A screening method for identifying an antalgic compound without any constipating and/or respiratory depressive effect comprising the following steps:
a) identifying an activator of a potassium channel selected from TREK-1, TRAAK and TREK-2 by screening candidate compounds;
b) measuring the antalgic effect of said activator; and
c) measuring the constipating and/or respiratory depressive effect of said activator.

4. The screening method according to claim 3, further comprising the step of selecting an antalgic compound without any constipating and/or respiratory depressive effect.

5. The use of a potassium channel selected from TREK-1, TRAAK and TREK-2 as a target for identifying an antalgic compound without any constipating and/or respiratory depressive effect during screening of candidate compounds.

6. The use according to claim 5, wherein said antalgic compound is an activator of said potassium channel.

7. The screening method according to any of claims 1 to 4, or the use according to claim 5 or 6, wherein said candidate compounds are selected from natural ligands of said potassium channel, chemical molecules, aptamers, peptides and antibodies.


### SEQUENCE LISTING

<110> UNIVERSITE D'AUVERGNE CLERMONT I
<120> The use of potassium channel activators TREK-1, TREK-2 or TRAAR as antalgies
<130> BFF 09A0386
<160> 3
<170> PatentIn version 3.4
<210> 1
   <211> 426
   <212> PRT
   <213> Homo sapiens
<220>
   <221> VARSPLIC
   <222> (1)..(16)
   <223> MLPSASRERPGYRAGV -> MMNPRAKRDFYL (in isoform 3)
<220>
   <221> VARSPLIC
   <222> (2)..(16)
   <223> Missing (in isoform_2)
<400> 1
   Met Leu Pro Ser Ala Ser Arg Glu Arg Pro Gly Tyr Arg Ala Gly Val
   1               5                   10                  15
   Ala Ala Pro Asp Leu Leu Asp Pro Lys Ser Ala Ala Gln Asn Ser Lys
               20                  25                  30
   Pro Arg Leu Ser Phe Ser Thr Lys Pro Thr Val Leu Ala Ser Arg Val
           35                  40                  45
   Glu Ser Asp Thr Thr Ile Asn Val Met Lys Trp Lys Thr Val Ser Thr
       50                  55                  60
   Ile Phe Leu Val Val Val Leu Tyr Leu Ile Ile Gly Ala Thr Val Phe
   65                  70                  75                  80
   Lys Ala Leu Glu Gln Pro His Glu Ile Ser Gln Arg Thr Thr Ile Val
                   85                  90                  95
   Ile Gln Lys Gln Thr Phe Ile Ser Gln His Ser Cys Val Asn Ser Thr
               100                 105                 110
   Glu Leu Asp Glu Leu Ile Gln Gln Ile Val Ala Ala Ile Asn Ala Gly
           115                 120                 125
   Ile Ile Pro Leu Gly Asn Thr Ser Asn Gln Ile Ser His Trp Asp Leu
       130                 135                 140
   Gly Ser Ser Phe Phe Phe Ala Gly Thr Val Ile Thr Thr Ile Gly Phe
   145 150 155 160
   Gly Asn Ile Ser Pro Arg Thr Glu Gly Gly Lys Ile Phe Cys Ile Ile
   165 170 175
   Tyr Ala Leu Leu Gly Ile Pro Leu Phe Gly Phe Leu Leu Ala Gly Val
   180 185 190
   Gly Asp Gln Leu Gly Thr Ile Phe Gly Lys Gly Ile Ala Lys Val Glu
   195 200 205
   Asp Thr Phe Ile Lys Trp Asn Val Ser Gln Thr Lys Ile Arg Ile Ile
   210 215 220
   Ser Thr Ile Ile Phe Ile Leu Phe Gly Cys Val Leu Phe Val Ala Leu
   225 230 235 240
   Pro Ala Ile Ile Phe Lys His Ile Glu Gly Trp Ser Ala Leu Asp Ala
   245 250 255
   Ile Tyr Phe Val Val Ile Thr Leu Thr Thr Ile Gly Phe Gly Asp Tyr
   260 265 270
   Val Ala Gly Gly Ser Asp Ile Glu Tyr Leu Asp Phe Tyr Lys Pro Val
   275 280 285
   Val Trp Phe Trp Ile Leu Val Gly Leu Ala Tyr Phe Ala Ala Val Leu
   290 295 300
   Ser Met Ile Gly Asp Trp Leu Arg Val Ile Ser Lys Lys Thr Lys Glu
   305 310 315 320
   Glu Val Gly Glu Phe Arg Ala His Ala Ala Glu Trp Thr Ala Asn Val
   325 330 335
   Thr Ala Glu Phe Lys Glu Thr Arg Arg Arg Leu Ser Val Glu Ile Tyr
   340 345 350
   Asp Lys Phe Gln Arg Ala Thr Ser Ile Lys Arg Lys Leu Ser Ala Glu
   355 360 365
   Leu Ala Gly Asn His Asn Gln Glu Leu Thr Pro Cys Arg Arg Thr Leu
   370 375 380
   Ser Val Asn His Leu Thr Ser Glu Arg Asp Val Leu Pro Pro Leu Leu
   385 390 395 400
   Lys Thr Glu Ser Ile Tyr Leu Asn Gly Leu Thr Pro His Cys Ala Gly
   405 410 415
   Glu Glu Ile Ala Val Ile Glu Asn Ile Lys
   420 425
<210> 2
   <211> 538
   <212> PRT
   <213> Homo sapiens
<220>
   <221> VARSPLIC
   <222> (1)..(12)
   <223> MFFLYTDFFLSL -> MKGDRTEGCRSDS (in isoform B)
<220>
   <221> VARSPLIC
   <222> (1)..(12)
   <223> MFFLYTDFFLSL -> MKFPIETPRKQVNWDPK (in isoform C)
<220>
   <221> VARIANT
   <222> (512)..(512)
   <223> A to T substitution
<400> 2
   Met Phe Phe Leu Tyr Thr Asp Phe Phe Leu Ser Leu Val Ala Val Pro
   1               5                   10                  15
   Ala Ala Ala Pro Val Cys Gln Pro Lys Ser Ala Thr Asn Gly Gln Pro
               20                  25                  30
   Pro Ala Pro Ala Pro Thr Pro Thr Pro Arg Leu Ser Ile Ser Ser Arg
           35                  40                  45
   Ala Thr Val Val Ala Arg Met Glu Gly Thr Ser Gln Gly Gly Leu Gln
       50                  55                  60
   Thr Val Met Lys Trp Lys Thr Val Val Ala Ile Phe Val Val Val Val
   65                  70                  75                  80
   Val Tyr Leu Val Thr Gly Gly Leu Val Phe Arg Ala Leu Glu Gln Pro
                   85                  90                  95
   Phe Glu Ser Ser Gln Lys Asn Thr Ile Ala Leu Glu Lys Ala Glu Phe
               100                 105                 110
   Leu Arg Asp His Val Cys Val Ser Pro Gln Glu Leu Glu Thr Leu Ile
           115                 120                 125
   Gln His Ala Leu Asp Ala Asp Asn Ala Gly Val Ser Pro Ile Gly Asn
   130 135 140
   Ser Ser Asn Asn Ser Ser His Trp Asp Leu Gly Ser Ala Phe Phe Phe
   145 150 155 160
   Ala Gly Thr Val Ile Thr Thr Ile Gly Tyr Gly Asn Ile Ala Pro Ser
   165 170 175
   Thr Glu Gly Gly Lys Ile Phe Cys Ile Leu Tyr Ala Ile Phe Gly Ile
   180 185 190
   Pro Leu Phe Gly Phe Leu Leu Ala Gly Ile Gly Asp Gln Leu Gly Thr
   195 200 205
   Ile Phe Gly Lys Ser Ile Ala Arg Val Glu Lys Val Phe Arg Lys Lys
   210 215 220
   Gln Val Ser Gln Thr Lys Ile Arg Val Ile Ser Thr Ile Leu Phe Ile
   225 230 235 240
   Leu Ala Gly Cys Ile Val Phe Val Thr Ile Pro Ala Val Ile Phe Lys
   245 250 255
   Tyr Ile Glu Gly Trp Thr Ala Leu Glu Ser Ile Tyr Phe Val Val Val
   260 265 270
   Thr Leu Thr Thr Val Gly Phe Gly Asp Phe Val Ala Gly Gly Asn Ala
   275 280 285
   Gly Ile Asn Tyr Arg Glu Trp Tyr Lys Pro Leu Val Trp Phe Trp Ile
   290 295 300
   Leu Val Gly Leu Ala Tyr Phe Ala Ala Val Leu Ser Met Ile Gly Asp
   305 310 315 320
   Trp Leu Arg Val Leu Ser Lys Lys Thr Lys Glu Glu Val Gly Glu Ile
   325 330 335
   Lys Ala His Ala Ala Glu Trp Lys Ala Asn Val Thr Ala Glu Phe Arg
   340 345 350
   Glu Thr Arg Arg Arg Leu Ser Val Glu Ile His Asp Lys Leu Gln Arg
   355 360 365
   Ala Ala Thr Ile Arg Ser Met Glu Arg Arg Arg Leu Gly Leu Asp Gln
   370 375 380
   Arg Ala His Ser Leu Asp Met Leu Ser Pro Glu Lys Arg Ser Val Phe
   385 390 395 400
   Ala Ala Leu Asp Thr Gly Arg Phe Lys Ala Ser Ser Gln Glu Ser Ile
   405 410 415
   Asn Asn Arg Pro Asn Asn Leu Arg Leu Lys Gly Pro Glu Gln Leu Asn
   420 425 430
   Lys His Gly Gln Gly Ala Ser Glu Asp Asn Ile Ile Asn Lys Phe Gly
   435 440 445
   Ser Thr Ser Arg Leu Thr Lys Arg Lys Asn Lys Asp Leu Lys Lys Thr
   450 455 460
   Leu Pro Glu Asp Val Gln Lys Ile Tyr Lys Thr Phe Arg Asn Tyr Ser
   465 470 475 480
   Leu Asp Glu Glu Lys Lys Glu Glu Glu Thr Glu Lys Met Cys Asn Ser
   485 490 495
   Asp Asn Ser Ser Thr Ala Met Leu Thr Asp Cys Ile Gln Gln His Ala
   500 505 510
   Glu Leu Glu Asn Gly Met Ile Pro Thr Asp Thr Lys Asp Arg Glu Pro
   515 520 525
   Glu Asn Asn Ser Leu Leu Glu Asp Arg Asn
   530 535
<210> 3
   <211> 393
   <212> PRT
   <213> Homo sapiens
<220>
   <221> VARSPLIC
   <222> (1)..(1)
   <223> M -> MTTAPQEPPARPLQAGSGAG PAPGRAM (in isoform 2)
<220>
   <221> VARIANT
   <222> (328)..(328)
   <223> P to L substitution
<400> 3